(19) **Europäisches Patentamt European Patent Office Office européen des brevets**

(11) **EP 1 242 027 B1**

(12) **EUROPEAN PATENT SPECIFICATION**

(45) Date of publication and mention
of the grant of the patent:
**25.07.2007 Bulletin 2007/30**

(51) Int Cl.:
**A61F 13/42** (2006.01)

(21) Application number: **99967251.2**

(22) Date of filing: **09.12.1999**

(86) International application number:
**PCT/US1999/029241**

(87) International publication number:
**WO 2001/041691 (14.06.2001 Gazette 2001/24)**

(54) **ABSORBENT ARTICLE WITH WETNESS INDICATOR**

SAUGFÄHIGER GEGENSTAND MIT FEUCHTIGKEITSANZEIGE

ARTICLE ABSORBANT AVEC INDICATEUR D'HUMIDITE

(84) Designated Contracting States:
**AT BE CH CY DE DK ES FI FR GB GR IE IT LI LU MC NL PT SE**

(43) Date of publication of application:
**25.09.2002 Bulletin 2002/39**

(73) Proprietor: **THE PROCTER & GAMBLE COMPANY Cincinnati, Ohio 45202 (US)**

(72) Inventors:
• **UEDA, Kimio**
**Higashinada-ku,**
**Kobe 658-0053 (JP)**

• **FURUSAWA, Shinichiro**
**Kobe 671-1602 (JP)**
• **KAWAKAMI, Yoshihisa**
**Higashinada-ku 658-0032 (JP)**

(74) Representative: **Borbach, Markus**
**Procter & Gamble Service GmbH,**
**Sulzbacher Strasse 40-50**
**65824 Schwalbach am Taunus (DE)**

(56) References cited:
**EP-A- 0 813 850         EP-A- 0 911 000**
**US-A- 5 817 076**

Printed by Jouve, 75001 PARIS (FR)

**Description**

TECHNICAL FIELD

This application relates to absorbent articles including, but not limited to, diapers, training pants, adult incontinence devices, diaper holders, feminine hygiene garments, and the like. More particularly, the present invention relates to absorbent articles having a wetness indicator used with a moisture pervious, liquid impervious back side cover, known for instance, from EP-A-0 813 850.

BACKGROUND

Absorbent articles, such as diapers, having a wetness indicator is known. The wetness indicator provides the wearer of the absorbent article and/or the caretaker with a visual signal when the absorbent article is wet by body discharges, such as urine, to let the wearer and/or the caretaker know whether or not a fresh pad is necessary. There have been many attempts for improvements of a wetness indicator, or of an absorbent article utilizing a wetness indicator.

Improvements in the use of a wetness indicator in absorbent articles are disclosed in, e.g., U.S. Patent 3,952,746 issued to Summers on April 27, 1976; U.S. Patent 4,022,211 issued to Timmons et al. on May 10, 1977; U.S. Patent 4,192,311 issued to Felfoldi on March 11, 1980; U.S. Patent 4,287,153 issued to Towsend on September 1, 1981; U.S. Patent 4,705,513 issued to Sheldon et al. on November 10, 1987; U.S. Patent 5,354,289 issued to Mitchell et al. on October 11, 1994; U.S. Patent 5,690,624 issued to Sasaki et al. on November 25, 1997; U.S. Patent 5,897,541 issued to Uitenbroek, et al. on April 27, 1999; Japanese Patent publication 91/221039 published on September 30, 1991; Japanese Patent publication 98/75980 published on March 24, 1998.

Improvements of wetness indicators are disclosed in, e.g., U.S. Patent 5,066,711 issued to Colon et al. on November 19, 1991; U.S. Patent 4,895,567 issued to Colon et al. on January 23, 1990; U.S. Patent 4,743,238 issued to Colon et al. on May 10, 1988; U.S. Patent 4,681,576 issued to Colon et al. on July 21, 1987; U.S. Patent 5,342,861 issued to Raykovitz on August 30, 1994.

Thus, there are many publications directed to improvements of a wetness indicator or improvements of an absorbent article utilizing a wetness indicator. However, none of these publications are directed to the use of a wetness indicator with a moisture pervious, liquid impervious backsheet (back side cover) for an absorbent article.

There have been attempts to improve the recognizability of a wetness indicator when used with a moisture pervious, liquid impervious backsheet in an absorbent article because the moisture pervious, liquid impervious sheet formed by stretching a plastic film containing an inorganic fillers typically presents opaqueness or translucency and obstructs the recognizability of a wetness indicator. Such technologies to improve the recognizability of a wetness indicator when used with a moisture pervious, liquid impervious sheet are disclosed in, e.g., U.S. Patent 5,766,212 issued to Jitoe et al. on June 16, 1998; and Japanese Patent publication 98/85257 published on April 7, 1998.

While the above publications are concerned with the recognizability of a wetness indicator through a moisture pervious, liquid impervious backsheet of an absorbent article, they are not concerned with a relation between the level of the sensitivity of a wetness indicator against moisture and the level of the moisture permeability of the backsheet. The wetness indicator is typically rendered to be readily active with liquids such that the wetness indicator is able to provide the wearer of the absorbent article and/or the caretaker with a visual signal. Due to the readiness of activation of the wetness indicator, while the wetness indicator provides a quick signal when wet, it detects even moisture in the air which may come through the moisture pervious, liquid impervious backsheet and tends to provide a wrong signal even though the wetness indicator is not wetted by body discharges, such as urine.

Thus, there is a need to provide an absorbent article with a wetness indicator used with a moisture pervious, liquid impervious back side cover. There is also a need to provide an absorbent article with a wetness indicator selected in relation to the moisture permeability of a moisture pervious, liquid impervious back side cover.

SUMMARY

The present invention is relevant to an absorbent article with a wetness indicator according to claim 1. The absorbent article comprises a liquid pervious topsheet, a moisture pervious, liquid impervious back side cover, an absorbent core interposed therebetween. The wetness indicator is disposed between the back side cover and the absorbent core. The moisture pervious, liquid impervious back side cover has a moisture vapor transmission rate of not less than 1,000 g/m$^2$/24hr. The wetness indicator remains visually inactive for at least 2 hours in the air of 85 % RH at 35 °C.

BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings in which:

FIG. 1 is a plan view of a disposable diaper embodiment of the present invention having portions cut away to reveal underlying structure, the inner surface of the diaper is facing the viewer;

FIG. 2 is a cross-sectional view of one embodiment of a disposable absorbent article shown in FIG. 1;

FIG. 3 is a partly enlarged cross sectional view of the portion of the dotted circle shown in FIG. 2 with one embodiment of a wetness indicator disposed in a disposable absorbent article; and

FIG. 4 is a plan view of a disposable diaper embodiment shown in FIG. 1 with the outer surface being faced with the viewer.

DETAILED DESCRIPTION

All cited references are incorporated herein by reference in their entireties. Citation of any reference is not an admission regarding any determination as to its availability as prior art to the claimed invention.

"Comprising" means that other steps and other elements which do not affect the end result can be added. This term encompasses the terms "consisting of" and "consisting essentially of".

All percentages, ratios and proportions used herein are by weight unless otherwise specified.

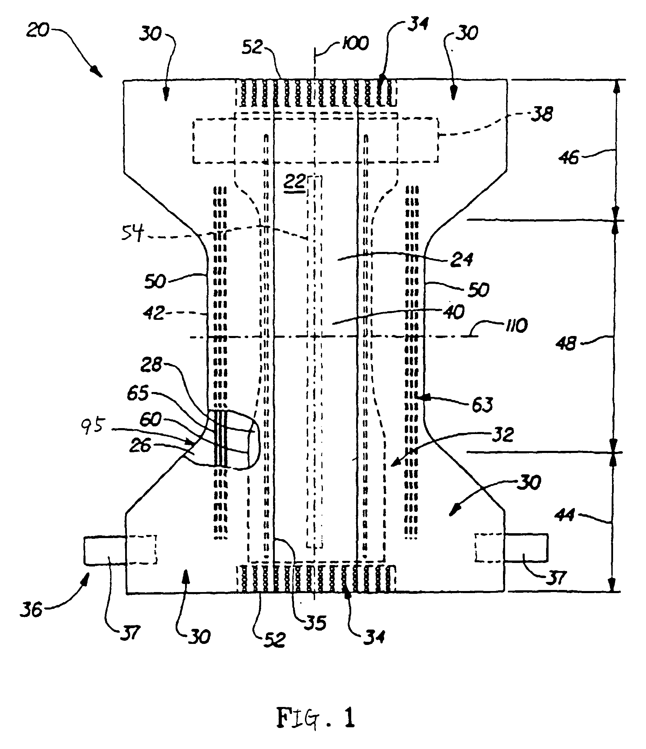

As used herein, the term "absorbent article" refers to devices which absorb and contain body exudates, and, more specifically, refers to devices which are placed against or in proximity to the body of the wearer to absorb and contain the various exudates discharged from the body. The term "disposable" is used herein to describe absorbent articles which are not intended to be laundered or otherwise restored or reused as an absorbent article (i.e., they are intended to be discarded after a single use and, preferably, to be recycled, composted or otherwise disposed of in an environmentally compatible manner). A preferred embodiment of an absorbent article of the present invention is the disposable absorbent article, diaper 20, shown in FIG. 1. As used herein, the term "diaper" refers to an absorbent article generally worn by infants and incontinent persons that is worn about the lower torso of the wearer. It should be understood, however, that the present invention is also applicable to other absorbent articles such as incontinence briefs, incontinence undergarments, diaper holders and liners, feminine hygiene garments, training pants, and the like.

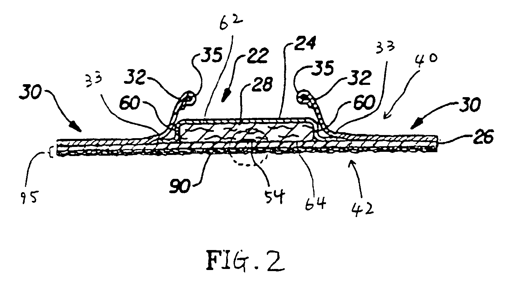

FIG. 1 is a plan view of the diaper 20 as one embodiment of the present invention in its flat-out, uncontracted state (i.e., with elastic induced contraction pulled out) with portions of the structure being cut-away to more clearly show the construction of the diaper 20 and with the portion of the diaper 20 which faces the wearer, the inner surface 40, facing the viewer. As shown in FIG. 1, the diaper 20 preferably comprises a containment assembly 22 comprising a liquid pervious topsheet 24; a moisture pervious, liquid impervious back side cover 95 joined to the topsheet; and an absorbent core 28 positioned between the topsheet 24 and the back side cover 95. The absorbent core 28 has a pair of opposing longitudinal edges 60, an inner surface 62 and an outer surface 64 (refer to FIG. 2). The diaper also comprises a wetness indicator 54 positioned between the moisture pervious, liquid impervious back side cover 95 and the absorbent core 28. The diaper preferably further comprises side panels 30; elasticized leg cuffs 32; elasticized waistbands 34; and a fastening system 36 preferably comprising a pair of securement members 37 and a landing member 38.

The diaper 20 is shown in FIG. 1 to have an inner surface 40 (facing the viewer in FIG. 1), an outer surface 42 opposed to the inner surface 40, a rear waist region 44, a front waist region 46 opposed to the rear waist region 44, a crotch region 48 positioned between the rear waist region 44 and the front waist region 46, and a periphery which is defined by the outer perimeter or edges of the diaper 20 in which the side edges are designated 50 and the end edges are designated 52. The inner surface 40 of the diaper 20 comprises that portion of the diaper 20 which is positioned adjacent to the wearer's body during use (i.e., the inner surface 40 generally is formed by at least a portion of the topsheet 24 and other components joined to the topsheet 24). The outer surface 42 comprises that portion of the diaper 20 which is positioned away from the wearer's body (i.e., the outer surface 42 is generally formed by at least a portion of the back side cover 95 and other components joined to the back side cover 95). As used herein, the term "joined" encompasses configurations whereby an element is directly secured to the other element by affixing the element directly to the other element, and configurations whereby the element is indirectly secured to the other element by affixing the element to intermediate member(s) which in turn are affixed to the other element. The rear waist region 44 and the front waist region 46 extend from the end edges 52 of the periphery to the crotch region 48.

The diaper 20 also has two centerlines, a longitudinal centerline 100 and a transverse centerline 110. The term "longitudinal", as used herein, refers to a line, axis, or direction in the plane of the diaper 20 that is generally aligned with (e.g. approximately parallel with) a vertical plane which bisects a standing wearer into left and right halves when the diaper 20 is worn. The terms "transverse" and "lateral", as used herein, are interchangeable and refer to a line, axis or direction which lies within the plane of the diaper that is generally perpendicular to the longitudinal direction (which divides the wearer into front and back body halves).

The containment assembly 22 of the diaper 20 is shown in FIG. 1 as comprising the main body (chassis) of the diaper 20. The containment assembly 22 preferably comprises a topsheet 24, a back side cover 95 and an absorbent core 28 having a pair of opposing longitudinal edges 60, an inner surface 62, an outer surface 64. The inner surface 62 generally faces the body of the wearer while the outer surface 64 generally faces away from the body of the wearer. When the absorbent article comprises a separate holder and a liner, the containment assembly 22 generally comprises the holder and the liner (i.e., the containment assembly 22 comprises one or more layers of material to define the holder while the liner comprises an absorbent composite such as a topsheet, a back side cover, and an absorbent core.) For unitary absorbent articles, the containment assembly 22 preferably comprises the topsheet 24, the back side cover 95 and the absorbent core 28 of the diaper with other features added to form the composite diaper structure.

FIG. 1 shows a preferred embodiment of the containment assembly 22 in which the topsheet 24 and the back side cover 95 have length and width dimensions generally larger than those of the absorbent core 28. The topsheet 24 and the back side cover 95 extend beyond the edges of the absorbent core 28 to thereby form the periphery of the diaper 20. While the topsheet 24, the back side cover 95, and the absorbent core 28 may be assembled in a variety of well known configurations, exemplary containment assembly configurations are described generally in U.S. Patent 3,860,003 entitled "Contractible Side Portions for Disposable Diaper" which issued to Kenneth B. Buell on January 14, 1975; and U.S. Patent 5,151,092 entitled "Absorbent Article With Dynamic Elastic Waist Feature Having A Predisposed Resilient Flexural Hinge" which issued to Kenneth B. Buell et al., on September 29, 1992; each of which is incorporated herein by reference.

The absorbent core 28 may be any absorbent member which is generally compressible, conformable, non-irritating to the wearer's skin, and capable of absorbing and retaining liquids such as urine and other certain body exudates. The absorbent core 28 may be manufactured in a wide variety of sizes and shapes (e.g., rectangular, hourglass, "T"-shaped, asymmetric, etc.) and from a wide variety of liquid-absorbent materials commonly used in disposable diapers and other absorbent articles such as comminuted wood pulp which is generally referred to as airfelt. Examples of other suitable absorbent materials include creped cellulose wadding; meltblown polymers including coform; chemically stiffened, modified or cross-linked cellulosic fibers; tissue including tissue wraps and tissue laminates; absorbent foams; absorbent sponges; superabsorbent polymers; absorbent gelling materials; or any equivalent material or combinations of materials.

The configuration and construction of the absorbent core 28 may vary (e.g., the absorbent core may have varying caliper zones, a hydrophilic gradient, a superabsorbent gradient, or lower average density and lower average basis weight acquisition zones; or may comprise one or more layers or structures). Further, the size and absorbent capacity of the absorbent core 28 may also be varied to accommodate wearers ranging from infants through adults. However, the total absorbent capacity of the absorbent core 28 should be compatible with the design loading and the intended use of the diaper 20.

One embodiment of the diaper 20 has an asymmetric, modified T-shaped absorbent core 28 having ears in the front waist region but a generally rectangular shape in the rear waist region. Exemplary absorbent structures for use as the absorbent core 28 of the present invention that have achieved wide acceptance and commercial success are described in U.S. Patent 4,610,678 entitled "High-Density Absorbent Structures" issued to Weisman et al. on September 9, 1986; U.S. Patent 4,673,402 entitled "Absorbent Articles With Dual-Layered Cores" issued to Weisman et al. on June 16, 1987; U.S. Patent 4,888,231 entitled "Absorbent Core Having A Dusting Layer" issued to Angstadt on December 19, 1989; and U.S. Patent 4,834,735, entitled "High Density Absorbent Members Having Lower Density and Lower Basis Weight Acquisition Zones", issued to Alemany et al. on May 30, 1989. The absorbent core may further comprise the dual core system containing an acquisition/distribution core of chemically stiffened fibers positioned over an absorbent storage core as detailed in U.S. Patent 5,234,423, entitled "Absorbent Article With Elastic Waist Feature and Enhanced Absorbency" issued to Alemany et al., on August 10, 1993; and in U.S. Patent 5,147,345, entitled "High Efficiency Absorbent Articles For Incontinence Management" issued to Young, LaVon and Taylor on September 15, 1992. All of these patents are incorporated herein by reference.

The topsheet 24 is preferably positioned adjacent the inner surface 62 of the absorbent core 28 and is preferably joined thereto and to the back side cover 95 by attachment means (not shown) such as those well known in the art. Suitable attachment means are described with respect to joining the back side cover 95 to the absorbent core 28. In a preferred embodiment of the present invention, the topsheet 24 and the back side cover 95 are joined directly to each other in the diaper periphery and are indirectly joined together by directly joining them to the absorbent core 28 by any suitable attachment means.

The topsheet 24 is preferably compliant, soft feeling, and non-irritating to the wearer's skin. Further, the topsheet 24 is preferably liquid pervious permitting liquids (e.g., urine) to readily penetrate through its thickness. A suitable topsheet 24 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers. The topsheet 24 is preferably made of a hydrophobic material to isolate the wearer's skin from liquids which have passed through the topsheet 24 and are contained in the absorbent core 28 (i.e. to prevent rewet). If the topsheet 24 is made of a hydrophobic material, at least the upper surface of the topsheet 24 is treated to be hydrophilic so that liquids will transfer through the topsheet more rapidly. This diminishes the likelihood that body exudates will flow off the topsheet 24 rather than being drawn through the topsheet 24 and being absorbed by the absorbent core 28. The topsheet 24 can be rendered hydrophilic by treating it with a surfactant. Suitable methods for treating the topsheet 24 with a surfactant include spraying the topsheet 24 material with the surfactant and immersing the material into the surfactant. A more detailed discussion of such a treatment and hydrophilicity is contained in U.S. Patents 4,988,344 entitled "Absorbent Articles with Multiple Layer Absorbent Layers" issued to Reising, et al on January 29, 1991 and U.S. Patent 4,988,345 entitled "Absorbent Articles with Rapid Acquiring Absorbent Cores" issued to Reising on January 29, 1991, each of which is incorporated by reference herein.

An alternative preferred topsheet comprises an apertured formed film. Apertured formed films are preferred for the topsheet because they are pervious to body exudates and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer. Suitable formed films are described in U.S. Patent 3,929,135, entitled "Absorptive Structures Having Tapered Capillaries", which issued to Thompson on December 30, 1975; U.S. Patent 4,324,246 entitled "Disposable Absorbent Article Having A Stain Resistant Topsheet", which issued to Mullane, et al. on April 13, 1982; U.S. Patent 4,342,314 entitled "Resilient Plastic Web Exhibiting Fiber-Like Properties", which issued to Radel. et al. on August 3, 1982; U.S. Patent 4,463,045 entitled "Macroscopically Expanded Three-Dimensional Plastic Web Exhibiting Non-Glossy Visible Surface and Cloth-Like Tactile Impression", which issued to Ahr et al. on July 31, 1984; and U.S. 5,006,394 "Multilayer Polymeric Film" issued to Baird on April 9, 1991. Each of these patents are incorporated herein by reference.

The back side cover 95 is that portion of the diaper 20 which is generally positioned away from the wearer's skin and which prevents the exudates absorbed and contained in the absorbent core 28 from wetting articles which contact the diaper 20 such as bedsheets and undergarments. Thus, the back side cover 95 is impervious to liquids (e.g., urine) and is preferably manufactured from a thin plastic film, although other flexible liquid impervious materials may also be used. (As used herein, the term "flexible" refers to materials which are compliant and will readily conform to the general shape and contours of the human body.) While the back side cover 95 is impervious to liquids, the back side cover 95 permits moisture to escape from the diaper 20.

The moisture vapor transmission rate of the back side cover 95 is important in reducing the incidence of heat rash and other skin problems associated with high heat humidity conditions. In order to reduce humidity and heat humidity within the diaper, the back side cover 95 has a weighed average moisture vapor transmission rate of not less than about 1,000 $g/m^2/24hr$, preferably not less than about 2,000 $g/m^2/24hr$, more preferably not less than about 2,500 $g/m^2/24hr$. While the upper end of the moisture vapor transmission rate depends on a type of a material, and is selected in relation to the liquid impermeability/dampness of the back side cover, the moisture vapor transmission rate may be not more than about 10,000 $g/m^2/24hr$.

The moisture vapor transmission rate is measured by the method set forth below. A known amount of $CaCl_2$ is put into a flanged cup. A sample is placed on the top of the cup and held securely by a retaining ring and gasket. The assembly is then weighed and recorded as the initial weight. The assembly is placed in a constant temperature (40°C) and humidity (75% RH (Relative Humidity)) chamber for 5 hours. The assembly is then removed from the chamber and allowed to equilibrate for at least 30 minutes at the temperature of the room where the balance is located. The assembly is then weighed and recorded as the final weight. The moisture vapor transmission rate (MVTR) is calculated and expressed in $g/m^2/24hr$ using the following formula.

$$MVTR = \frac{(\text{Final weight - Initial weight}) \times 24}{\text{Area of sample in meters} \times 5 \text{ (time in chamber)}}$$

The back side cover 95 of the present invention may comprise a single layer material such as a film, or may comprise two or more layers of material joined together to form a laminate of the back side cover 95. For example, the back side cover 95 may comprise a single film which is moisture pervious and liquid impervious. Alternatively, the back side cover 95 may comprise a laminate comprising a moisture pervious, liquid impervious film and a moisture pervious outer sheet joined to the film. If the back side cover 95 comprises a single film, the sample in the measurement of MVTR described above should be the film. If the back side cover 95 comprises a laminate, the sample in the measurement of MVTR should be the laminate.

The back side cover 95 is preferably positioned adjacent the outer surface 64 of the absorbent core 28 and is preferably joined thereto by any suitable attachment means known in the art. For example, the back side cover 95 may be secured to the absorbent core 28 by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota and marketed as HL-1258. An example of a suitable attachment means comprising an open pattern network of filaments of adhesive is disclosed in U.S. Patent 4,573,986 entitled "Disposable Waste-Containment Garment", which issued to Minetola et al. on March 4, 1986. Another suitable attachment means comprising several lines of adhesive filaments swirled into a spiral pattern is illustrated by the apparatus and methods shown in U.S. Patent 3,911,173 issued to Sprague, Jr. on October 7, 1975; U.S. Patent 4,785,996 issued to Ziecker, et al. on November 22, 1978; and U.S. Patent 4,842,666 issued to Werenicz on June 27, 1989. Each of these patents are incorporated herein by reference. Alternatively, the attachment means may comprise heat bonds, pressure bonds, ultrasonic bonds, dynamic mechanical bonds, or any other suitable attachment means or combinations of these attachment means as are known in the art. Embodiments of the present invention are also contemplated wherein the absorbent core is not joined to the back side cover 95, and/or the topsheet 24 in order to provide greater extensibility in the front waist region 46 and the rear waist region 44.

In the embodiment shown in FIGS. 1 and 2, the back side cover 95 comprises a backsheet film 26 which may comprise a breathable microporous film and an outer sheet 90 which may comprise a nonwoven. The backsheet film 26 positions adjacent the absorbent core 28 such that the backsheet film 26 faces the absorbent core 28. Alternatively, another layer of material may be inserted between the absorbent core 28 and the backsheet film 26. The outer sheet 90 positions outwardly of the diaper.

The backsheet film 26 may comprise any known material being moisture pervious and liquid impervious. For example, the backsheet film 26 may comprise a breathable microporous film composed of a thermoplastic resin and inorganic fillers dispersed in the thermoplastic resin. Suitable thermoplastic polymers include polyolefins such as polyethylenes, including liner low density polyethylene (LLDPE), low density polyethylene (LDPE), ultra low density polyethylene (ULDPE), high density polyethylene (HDPE), or polypropylene and blends thereof with the above and other materials. Examples of other suitable thermoplastic polymers which may also be used include, but are not limited to, polyester, polyurethanes, compostable or biodegradable polymers, thermoplastic elastomers, and metallocene catalyst-based polymers (e.g., INSITE® available from Dow Chemical Company and Exxact® available from Exxon). The inorganic material or filler is selected from the group consisting of calcium carbonate, clay and titanium dioxide, with the preferred inorganic filler being calcium carbonate. The inorganic filler may be coated with a fatty acid ester to obtain higher loadings in the polymer. The inorganic filler and the thermoplastic polymer are blended together to form a homogeneous mixture in a suitable mixing extruder, or in a separate preliminary compounding step. The mixture is then cast or blown into a film. The obtained film is stretched at least in one direction to impart breathability on the substantially entire area of the film. The step of stretching a film to impart breathability may be done at a different place prior to manufacturing process of absorbent articles. Alternatively, the step of stretching may be done at the same place, i.e., same manufacturing process, prior to assembling a breathable microporous film with other elements of absorbent articles. In any case, the film is imparted breathability on the substantially entire area of the film before the resulting breathable microporous film is assembled with other elements of absorbent articles. Suitable backsheet film is supplied from Mitsui Chemical, Japan under the name of Espoir.

The back side cover 95 may further comprise an outer sheet 90 joined with at least a portion of the garment-facing surface of the backsheet film 26. The outer sheet 90 preferably comprises a nonwoven web (outer nonwoven sheet). (However, embodiments are contemplated wherein the outer sheet 90 comprises materials such as woven webs, foams, scrims, films, loose fibers, or any other material or combination of materials known in the art that will give the diaper a cloth-like look and/or feel and is at a minimum air permeable.) The outer sheet 90 may cover all or substantially all of the garment-facing surface of the backsheet film 26, or may cover only discrete predetermined portions. In a preferred embodiment, the nonwoven web of the outer sheet 90 covers all or substantially all of the backsheet film 26 in order to provide the diaper with a cloth-like look and feel. Further, the outer sheet 90 may provide the diaper with a low cost landing zone capable of engaging the hooks of a hook and loop type fastener. (Such a landing zone could be utilized as a portion of a primary fastening system or as a means for disposing of a soiled diaper.)

The nonwoven web comprised in the outer sheet 90 may comprise natural fibers (e.g. cotton or wood fibers), or may comprise fibers of polyethylene, polypropylene, polyester, or any combination of such fibers. Further, the nonwoven may be carded, spunmelt, meltblown or air-through bonded or have any other characteristic or be manufactured in any manner known in the art. Preferably, the nonwoven is comprised of sufficient thermoplastic material to allow for thermal bonding of the material to other components of the diaper. An especially preferred nonwoven is a carded nonwoven made of 100% polypropylene fibers such as FPN 290 manufactured by FiberWeb North America of Simpsonville, SC.

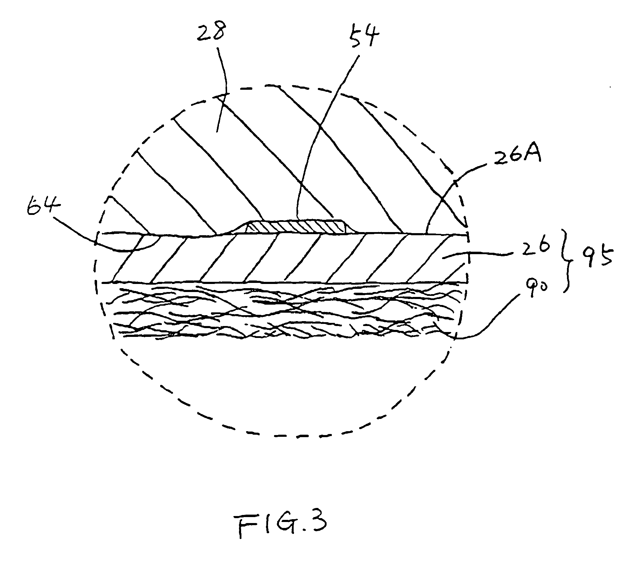

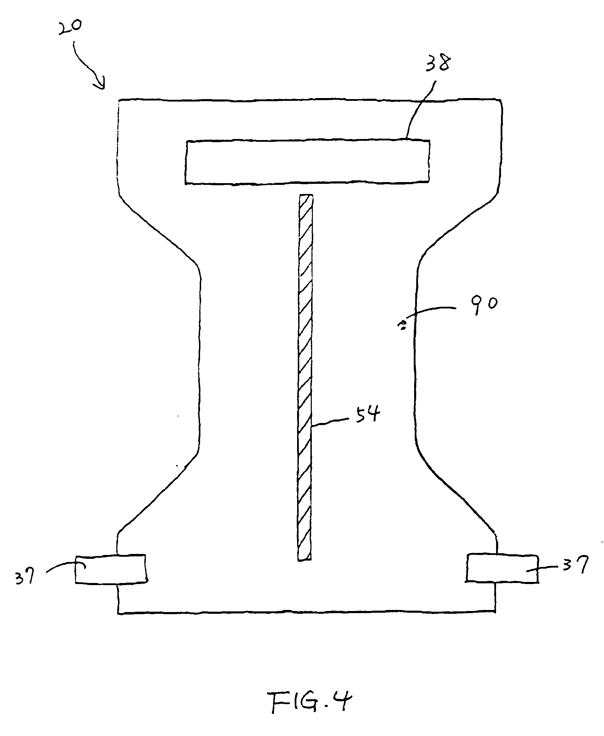

The diaper 20 comprises a wetness indicator 54. The wetness indicator 54 positions between the moisture pervious, liquid impervious back side cover 95 and the absorbent core 28. In the embodiment, the back side cover 95 comprises the backsheet film 26 which faces outer surface 64 of the absorbent core 28 and the outer sheet 90 which faces outwardly of the diaper 20. The wetness indicator 54 is disposed on the inner surface 26A of the backsheet film 26 and the outer surface 28A of the absorbent core 28 as shown in FIG. 3. The wetness indicator 54 may comprise a single stripe as shown in FIGS. 1 and 4, alternatively may comprise two or more stripes. The wetness indicator 54 extends along only a portion of the longitudinal centerline of the diaper 20 as shown in FIGS. 1 and 4, alternatively may extend along the entire longitudinal centerline of the diaper 20. The wetness indicator may have any shape, such as dot, circle, triangle, rectangle, decorative indicia, letter, etc. as far as the wetness indicator provides a visual signal when the diaper is worn and wetted.

The wetness indicator 54 provides a visual signal which can be seen through the back side cover 95 when wetted. The wetness indicator 54 may have a visible color before the wetness indicator 54 is wetted so that the presence of the wetness indicator 54 is visible from the outside. If the wetness indicator 54 is visible before wetted, the wetness indicator 54 may change the color to a different color when wet to provide a visual signal. Alternatively, the visible color of the wetness indicator 54 may disappear when the wetness indicator 54 is wetted. The wetness indicator 54 may be invisible before the wetness indicator 54 is wetted. If the wetness indicator 54 is invisible before wetted, the wetness indicator 54 may appear when wet.

Preferably, the wetness indicator is readily active with liquids such that the wetness indicator is able to provide the wearer of the absorbent article and/or the caretaker with a visual signal when the wetness indicator is wetted by liquids. However, preferably the wetness indicator does not become readily active with moisture in the air which may come through the moisture pervious, liquid impervious back side cover to avoid providing a wrong signal to the wearer of the absorbent article and/or the caretaker. Therefore, the level of the sensitivity of the wetness indicator is important when the wetness indicator is used with a moisture pervious, liquid impervious back side cover in an absorbent article such as a disposable diaper.

The sensitivity of the wetness indicator may be described in two aspects. One is the sensitivity of the wetness indicator under the presence of moisture. The other is the sensitivity of the wetness indicator under the presence of liquid, such as urine. The sensitivity under the presence of moisture is particularly important in the use with a moisture pervious, liquid impervious back side cover in an absorbent article such that the wetness indicator does not become readily active by moisture which comes through the moisture pervious back side cover. The sensitivity of the wetness indicator under the presence of moisture is determined by a visual change of the wetness indicator under a specific environment. The wetness indicator of the present invention remains visually inactive for at least 2 hours in the air of 85 % RH at 35 °C, preferably for at least 8 hours in the air of 85 % RH at 35 °C, more preferably for at least 18 hours in the air of 85 % RH at 35 °C. If the wetness indicator becomes visually active before 2 hours in the air of 85 % RH at 35 °C, such a wetness indicator may not be used with a moisture pervious, liquid impervious back side cover which has a moisture vapor transmission rate of not less than about 1,000 $g/m^2/24hr$. The wetness indicator may remain visually inactive for at least 5 hours in the air of 70 % RH at 30 °C, preferably for at least 12 hours in the air of 70 % RH at 30 °C, more preferably for at least 20 hours in the air of 70 % RH at 30 °C. Further, the wetness indicator may remain visually inactive for at least 10 hours in the air of 90 % RH at 20 °C, preferably for at least 24 hours in the air of 90 % RH at 20 °C, more preferably for at least 48 hours in the air of 90 % RH at 20 °C.

The sensitivity of the wetness indicator under the presence of moisture is measured by the method set forth below. A fresh diaper is taken out from a package of the diapers. The fresh diaper is placed in a constant temperature (35 °C) and humidity (85 % RH) oven without unfolding the fresh diaper such that the wetness indicator of the diaper is exposed to the air in the oven. Then, the visual change of the wetness indicator is observed every hour. For the observation every hour, the diaper is taken out from the oven for 30 seconds to observe a visual change of the wetness indicator. The visual change of the wetness indicator is observed by naked normal eyes with a distance of 50 cm. If the visual change, such as color change or appearance/disappearance, is observed on the wetness indicator, it is assessed that the wetness indicator became visually active at this time of observation, but the wetness indicator remained visually inactive until the last time of observation. After the observation, the diaper is returned in the oven until the next observation. If the wetness indicator changes the color (i.e., from one visible color to another visible color) for a wetness signal, the visual change of the wetness indicator is determined by the observer's first recognition of the color change. For example, if the wetness indicator changes its color from yellow to blue through 4 phases; yellowish green, greenish yellow, green, and greenish blue in its order, the observer's first recognition of the color change from yellow to yellowish green is noted as a visual change. If the wetness indicator appears for a wetness signal (i.e., wetness indicator is invisible in the inactive state, but becomes visible in the active state), the visual change of the wetness indicator is determined by the observer's first recognition of the appearance of the wetness indicator. If the wetness indicator disappears for a wetness signal (i.e., wetness indicator is visible in the inactive state, but becomes invisible in the active state), the visual change is determined by the observer's first recognition of the decrease of color intensity of the wetness indicator.

The sensitivity of the wetness indicator under the presence of liquid, such as urine is determined by the pH range in which the wetness indicator becomes visually active. The wetness indicator of the present invention becomes visually active in the pH range of at least 4, preferably at least 4.5, more preferably at least 5. As far as the wetness indicator becomes active at least 4, the upper end of the pH range is arbitrary while it is limited by pH of 14. Preferably, the upper end of the pH range may be limited by pH of 8.

The pH range in which the wetness indicator becomes visually active is measured by the method set forth below. A pH solution adjusted to be a predetermined pH (e.g. pH of 4) is prepared. A fresh diaper is taken out from a package of the diapers. The adjusted pH solution of 40 ml is loaded on the side of the topsheet of the fresh diaper in the area where the wetness indicator is present. After waiting for one (1) minute, the visual change of the wetness indicator is observed from the outside of the diaper (i.e., from the side of the back side cover) by naked normal eyes with a distance of 50 cm. If the visual change, such as color change, is observed on the wetness indicator, it is assessed that the wetness indicator became visually active by the predetermined pH.

The wetness indicator is provided on the absorbent article in the basis weight range of between 1 $g/m^2$ and 100 $g/m^2$, preferably between 3 $g/m^2$ and 70 $g/m^2$, more preferably between 5 $g/m^2$ and 50 $g/m^2$.

The wetness indicator comprises a base polymer as a main ingredient. The base polymer preferably inhibits the color forming reaction in the absence of water in the wetness indicator and preferably inhibits, at least desensitizes the color forming reaction in the presence of moisture in the wetness indicator. The base polymer is preferably relatively hydrophobic. Suitable base polymer for use in the present invention ranges in molecular weight of more than about 2,000, preferably more than about 5,000, and more preferably more than about 10,000. If the molecular weight of the base polymer is less than about 2,000, the wetness indicator is a solution or wax state, and therefore the moisture in the air easily resolves in the wetness indicator, thereby changing the color of the wetness indicator.

The wetness indicator of the present invention contains from about 20 to about 99 wt. % of base polymer, preferably from about 30 to about 95 wt. % of base polymer, more preferably from about 40 to about 90 wt. % of base polymer. If the wetness indicator contains less than about 20 % of base polymer, the wetness indicator will have insufficient hydrophobicity to inhibit, at least to desensitize the color forming reaction in the presence of moisture in the air. If the wetness indicator contains more than about 99 % of base polymer, the wetness indicator will be too much hydrophobic to make the color forming reaction in the presence of the liquid such as urine.

Preferred examples of base polymer for use in the present invention include; polyethylene glycol, such as CARBOXWAX sold by Union Carbide; polypropylene glycol, such as CoatSoam sold by Nippon Oil and Fat Company; polyethylene glycol-polypropylene glycol block copolymers, such as Pluronic®, and Pluronic R® surfactants sold by BASF-Wyandotte Corp.; ethoxylated branched aliphatic diols such as Surfynol® surfactants sold by Air Product & Chemicals, Inc.; ethoxylated aliphatic alcohols and carboxylic acids; polyethylene glycol diesters of fatty acids; polyalkyleneoxide polysiloxanes; and mixtures thereof.

Other preferred examples of base polymer for use in the present invention include glycerin; dodecylamine; 2,4,4-trimethyl-2-oxazoline; N,N-di(polyoxyethylene)ethylamine; polyoxypropylene-diethylamine adducts; nonionic surfactants such as polyoxyethylene lauryl ether, polyoxyethylene oleyl ether, polyoxyethylene nonyl phenyl ether, polyoxyethylene sorbitan monolaurate, polyethylene glycol monostearate or the like.

The wetness indicator also comprises a wetness indicating agent. The wetness indicating agent comprises a material which is capable of changing the color quickly when the wetness indicating agent is wet, and of course, the readily visible color must be easily distinguish from the color of the dry composition. The wetness indicator of the present invention contains from about 1 to about 80 wt. % of wetness indicting agent, preferably from about 5 to about 70 wt. % of wetness indicting agent, more preferably from about 10 to about 60 wt. % of wetness indicting agent. If the wetness indicator contains less than about 1 % of wetness indicating agent, it becomes difficult to observe the color change of wetness indicator. If the wetness indicator contains more than about 80 % of wetness indicating agent, the wetness indicator changes the color regardless of the presence of the base polymer which inhibits, at least to desensitizes the color forming reaction in the presence of moisture.

Acid-base wetness indicating agents, which change color in response to a change in pH, are preferred, because they change color rapidly. Preferred acid-base wetness indicating agents are those which change color at a pH in the range of over 3.5, such as Ethyl Red, Bromophenol Blue (made by Eastman Kodak), Bromocresol Green, or Resazurine. Other materials which change color in response to water may be used as the wetness indicating agent, such as dyes which are substantially invisible in the dry composition, but which quickly become a vivid color when wet. An example of such a material is the blue dye, Calcocid® Blue 2G, made by American Cyanamid Corporation.

The wetness indicator may comprise other components, e.g., to render the wetness indicator be a hot melt adhesive. For this purpose, the wetness indicator may contain tackifying resins and plasticizing oil and/or wax diluent.

The wetness indicator may contain one or more compatible tackifying resins in amounts from 0 wt. % up to about 50 wt. %. The tackifying resin employed in the compositions increases tack. The tackifying resin component appears to extend the adhesive properties, particular wetting ability, and viscosity control of the base polymer. The tackifying resin includes natural and modified rosins such as gum rosin, wood rosin, tall-oil rosin, distilled rosin, hydrogenated rosin, dimerized rosin, and polymerized rosin. Preferably, the tackifying resin is present in an amount ranging from about 10 to about 40 wt. % of the final composition.

The adhesive may also contain a compatible plasticizing oil and/or wax diluent in an amount from 0 wt. % up to 35 wt. %. Plasticizers have typically been employed to lower the viscosity of the overall adhesive composition without substantially decreasing the adhesive strength and/or the service temperature of the adhesive. Particularly useful is the Unithox 550® , a modified synthetic wax (melting point of 209° F.) available from Petrolite. Other useful diluents include Benzoflex 352® , a 1,4-cyclohexane dimethanol dibenzante from Velsicol Chemical Corporation and Pycal 94® , a phenyl ether of polyethylene glycol, from Atlas Powder. Preferably, the plasticizing resin is present in an amount ranging from about 20 to about 30 wt. % of the final composition.

A suitable wetness indicator comprising a base polymer, a wetness indicating agent, a tackifying resin, and a plasticizing oil, and having the form of a hot melt adhesive is provided by Ato Findley Inc., US, under the name of H9219.

The diaper 20 may have extensibility or elasticity in all or a portion of the side panels 30. (As used herein, the term "extensible" refers to materials that are capable of extending in at least one direction to a certain degree without undue rupture. The terms "elasticity" and "elastically extensible" refer to extensible materials that have the ability to return to approximately their original dimensions after the force that extended the material is removed. As used herein, any material or element described as "extensible" may also be elastically extensible unless otherwise provided.) Extensible side panels 30 provide a more comfortable and contouring fit by initially conformably fitting the diaper to the wearer and sustaining this fit throughout the time of wear well passed when the diaper has been loaded with exudates since the side panels allow the sides of the diaper to expand and contract. Extensible side panels 30 further provide more effective application of the diaper 20 since even if the diaperer pulls one side panel 30 farther than the other during the application (asymmetrically), the diaper 20 will "self-adjust" during wear. While the extensible side panels 30 may be constructed in a number of configurations, examples of diapers with extensible side panels are disclosed in U.S. Pat. No. 4,857,067, entitled "Disposable Diaper Having Shirred Ears" issued to Wood, et al. on August 15, 1989; U.S. Pat. No. 4,381,781 issued to Sciaraffa, et al. on May 3, 1983; U.S. Pat. No. 4,938,753 issued to Van Gompel, et al. on July 3, 1990; and in U.S. Pat. No. 5,151,092 issued to Buell et al. on September 29, 1992; each of which are incorporated herein by reference.

The diaper 20 further may comprise elasticized leg cuffs 32 for providing improved containment of liquids and other body exudates. Each elasticized leg cuff 32 may comprise several different embodiments for reducing the leakage of body exudates in the leg regions. Such embodiments are disclosed in, e.g., U.S. Patent 4,909,803 issued to Aziz et al. on March 20, 1990; U.S. Patent 4,695,278 issued to Lawson on September 22, 1987; U.S. Patent 4,795,454 issued to Dragoo on January 3, 1989; and U.S. Patent 4,704,115 issued to Buell on November 3, 1987; each of which are incorporated herein by reference.

It is preferred that each elasticized leg cuff 32 comprise at least an inner barrier cuff comprising a barrier flap and a spacing element such as described in the above-referenced U.S. Pat. No. 4,909,803. In a preferred embodiment, the elasticized leg cuff 32 additionally comprises an elastic gasketing cuff 63 with one or more elastic strands 65, positioned outboard of the barrier cuff such as described in the above-referred U.S. Pat. No. 4,695,278. Further, as shown in FIG. 2, the elasticized leg cuff 32 preferably has a proximal edge 33 and a distal edge 35. The distal edge 35 of the elasticized leg cuff 32 is that part of the elasticized leg cuff 32 which is spaced away from the chassis 22 of the diaper when the diaper 20 is being worn. The proximal edge 33 is that part of the elasticized leg cuff 32 which is joined to the chassis 22 of the diaper 20.

The diaper 20 further may comprise an elasticized waistband 34 that provides improved fit and containment. The elasticized waistband 34 is that portion or zone of the diaper 20 which is intended to elastically expand and contract to dynamically fit the wearer's waist. The elasticized waistband 34 preferably extends longitudinally outwardly from at least one of the waist edges of the absorbent core 28 and generally forms at least a portion of the end edge of the diaper 20. Disposable diapers are generally constructed so as to have two elasticized waistbands, one positioned in the rear waist region and one positioned in the front waist region, although diapers can be constructed with a single elasticized waistband. Further, while the elasticized waistband 34 or any of its constituent elements can comprise a separate element affixed to the diaper 20, the elasticized waistband 34 may be constructed as an extension of other elements of the diaper such as the backsheet 26 or the topsheet 24, preferably both the backsheet 26 and the topsheet 24. Embodiments are also contemplated wherein the elasticized waistband 34 comprises apertures, as described above, to provide breathability in the waist regions. The elasticized waistband 34 may be constructed in a number of different configurations including those described in U.S. Patent 4,515,595 entitled "Disposable Diapers with Elastically Contractible Waistbands" issued to Kievit et al. on May 7,1985 and the above referenced U.S. Patent 5,151,092 issued to Buell; each of these references being incorporated herein by reference.

The diaper 20 also comprises a fastening system 36 which forms a side closure which maintains the rear waist region 44 and the front waist region 46 in an overlapping configuration such that lateral tensions are maintained around the circumference of the diaper to maintain the diaper on the wearer. Exemplary fastening systems are disclosed in U.S. Pat. No. 3,848,594 issued to Buell on November 19, 1974; U.S. Pat. No. 4,662,875 issued to Hirotsu and Robertson on May 5, 1987; U.S. Pat. No. 4,869,724 issued to Scripps on September 26, 1989; U.S. Pat. No. 4,846,815 issued to Scripps on July 11, 1989; U.S. Pat. No. 4,894,060 issued to Nestegard on January 16, 1990; U.S. Pat. No. 4,946,527 issued to Battrell on August 7, 1990; and U.S. Patent 5,326,612 entitled "Nonwoven Female Component For Refastenable Fastening Device And Method of Making the Same" issued to David J. K. Goulait on July 5, 1994. Each of these patents are incorporated herein by reference.

It should also be understood that all of the limits and ranges specified herein include all narrower ranges, limits, and amounts that are within the specified limits and ranges.

Particular embodiments of the present invention have been illustrated and described.

## Claims

1. An absorbent article (20) with a wetness indicator, the absorbent article comprising a liquid pervious topsheet (24), a moisture pervious, liquid impervious back side cover (95), an absorbent core (28) interposed therebetween, the wetness indicator (54) disposed between the back side cover (25) and the absorbent core (28), wherein

   the moisture pervious, liquid impervious back side cover (95) has a moisture vapor transmission rate as defined herein of not less than 1,000 g/m$^2$/24hr,

   the wetness indicator (54) remains visually inactive as defined herein for at least 2 hours in the air of 85 % RH (relative humidity) at 35°C.

2. The absorbent article (20) of Claim 1 wherein the wetness indicator (54) remains visually inactive for at least 5 hours in the air of 70 % RH at 30 °C.

3. The absorbent article (20) of Claim 2 wherein the wetness indicator (54) remains visually inactive for at least 10 hours in the air of 90 % RH at 20 °C.

4. The absorbent article (20) of Claim 1 wherein the wetness indicator (54) becomes visually active in the pH range of at least 4.

5. The absorbent article (20) of Claim 1 wherein the wetness indicator (54) is applied in the basis weight range of between 1 g/m$^2$ and 100 g/m$^2$.

6. The absorbent article (20) of Claim 1 wherein the wetness indicator (54) comprises a base polymer and a wetness indicating agent

7. The absorbent article (20) of Claim 6 wherein the base polymer is selected from the group consisting of polyethylene glycol; polypropylene glycol; polyethylene glycol-polypropylene glycol block copolymers; ethoxylated branched aliphatic diols; ethoxylated aliphatic alcohols and carboxylic acids; polyethylene glycol diesters of fatty acids; polyalkyleneoxide polysiloxanes; or mixtures thereof.

8. The absorbent article (20) of Claim 7 wherein the base polymer is selected from the group consisting of polyethylene glycol; polypropylene glycol; polyethylene glycol-polypropylene glycol block copolymers; or mixtures thereof.

9. The absorbent article (20) of Claim 6 wherein the wetness indicating agent is selected from the group consisting of Ethyl Red, Bromophenol Blue, Bromocresol Green, or Resazurine.

10. The absorbent article (20) of Claim 6 wherein the wetness indicator (54) is in the form of a hot melt adhesive, and further includes a plasticizer and a tackifier.

## Patentansprüche

1. Absorptionsartikel (20) mit einem Nässeanzeiger, wobei der Absorptionsartikel eine flüssigkeitsdurchlässige Oberschicht (24), eine feuchtigkeitsdurchlässige, flüssigkeitsundurchlässige Rückseitenabdeckung (25), einen Absorptionskern (28), der dazwischen angeordnet ist, aufweist, der Nässeanzeiger (54) zwischen der Rückseitenabdeckung (25) und dem Absorptionskern (28) angeordnet ist, wobei

   die feuchtigkeitsdurchlässige, flüssigkeitsundurchlässige Rückseitenabdeckung (25) eine Wasserdampf-Übertragungsrate wie hierin definiert von nicht unter 1000 g/m$^2$/24 h aufweist,

   der Nässeanzeiger (54) für mindestens 2 Stunden an Luft mit einer RF (relativen Feuchtigkeit) von 85 % bei 35 °C visuell inaktiv bleibt, wie hierin definiert.

**2.** Absorptionsartikel (20) nach Anspruch 1, wobei der Nässeanzeiger (54) für mindestens 5 Stunden an Luft mit 70 % RF bei 30 °C visuell inaktiv bleibt.

**3.** Absorptionsartikel (20) nach Anspruch 2, wobei der Nässeanzeiger (54) für mindestens 10 Stunden an Luft mit 90 % RF bei 20 °C visuell inaktiv bleibt.

**4.** Absorptionsartikel (20) nach Anspruch 1, wobei der Nässeanzeiger (54) im pH-Bereich von mindestens 4 visuell aktiv wird.

**5.** Absorptionsartikel (20) nach Anspruch 1, wobei der Nässeanzeiger (54) im Flächengewichtsbereich zwischen 1 g/m$^2$ und 100 g/m$^2$ angewendet wird.

**6.** Absorptionsartikel (20) nach Anspruch 1, wobei der Nässeanzeiger (54) ein Basispolymer und ein Nässeanzeigemittel umfasst.

**7.** Absorptionsartikel (20) nach Anspruch 6, wobei das Basispolymer ausgewählt ist aus der Gruppe, bestehend aus Polyethylenglycol; Polypropylenglycol; Polyethylenglycol/Polypropylenglycol-Blockcopolymeren; ethoxylierten verzweigten aliphatischen Diolen; ethoxylierten aliphatischen Alkoholen und Carbonsäuren; Polyethylenglycoldiestern von Fettsäuren; Polyalkylenoxidpolysiloxanen oder Mischungen davon.

**8.** Absorptionsartikel (20) nach Anspruch 7, wobei das Basispolymer ausgewählt ist aus der Gruppe, bestehend aus Polyethylenglycol; Polypropylenglycol; Polyethylenglycol/Polypropylenglycol-Blockcopolymeren oder Mischungen davon.

**9.** Absorptionsartikel (20) nach Anspruch 6, wobei das Nässeanzeigemittel ausgewählt ist aus der Gruppe, bestehend aus Ethylrot, Bromphenolblau, Bromcresolgrün oder Resazurin.

**10.** Absorptionsartikel (20) nach Anspruch 6, wobei der Nässeanzeiger (54) in Form eines Schmelzklebers vorliegt und ferner einen Weichmacher und einen Haftvermittler einschließt.

**Revendications**

**1.** Article absorbant (20) avec un indicateur d'humidité, l'article absorbant comprenant une feuille de dessus perméable aux liquides (24), une protection arrière perméable à l'humidité, imperméable aux liquides (95), une âme absorbante (28) interposée là entre, l'indicateur d'humidité (54) disposé entre la protection arrière (95) et l'âme absorbante (28), dans lequel
la protection arrière perméable à l'humidité, imperméable aux liquides (95) a une vitesse de transmission de vapeur humide telle que définie ici de pas moins de 1000 g/m$^2$/24h,
l'indicateur d'humidité (54) reste visuellement inactif, tel que défini ici, pendant au moins 2 heures dans une atmosphère à 85 % d'humidité relative à 35 °C.

**2.** Article absorbant (20) selon la revendication 1, dans lequel l'indicateur d'humidité (54) reste visuellement inactif pendant au moins 5 heures dans une atmosphère à 70 % d'humidité relative à 30 °C.

**3.** Article absorbant (20) selon la revendication 2, dans lequel l'indicateur d'humidité (54) reste visuellement inactif pendant au moins 10 heures dans une atmosphère à 90 % d'humidité relative à 20 °C.

**4.** Article absorbant (20) selon la revendication 1, dans lequel l'indicateur d'humidité (54) devient visuellement actif dans la gamme de pH d'au moins 4.

**5.** Article absorbant (20) selon la revendication 1, dans lequel l'indicateur d'humidité (54) est appliqué dans l'intervalle de masse surfacique compris entre 1 g/m$^2$ et 100 g/m$^2$.

**6.** Article absorbant (20) selon la revendication 1, dans lequel l'indicateur d'humidité (54) comprend un polymère de base et un agent indiquant l'humidité.

**7.** Article absorbant (20) selon la revendication 6, dans lequel le polymère de base est choisi dans le groupe constitué de polyéthylène glycol ; polypropylène glycol ; copolymères séquencés de polyéthylène glycol-polypropylène glycol ; diols aliphatiques ramifiés éthoxylés; alcools aliphatiques éthoxylés et acides carboxyliques ; diesters de polyéthylène glycol d'acides gras ; polysiloxanes polyalkènes oxydes ; ou leurs mélanges.

**8.** Article absorbant (20) selon la revendication 7, dans lequel le polymère de base est choisi dans le groupe constitué de polyéthylène glycol ; polypropylène glycol ; copolymères séquencés de polyéthylène glycol-polypropylène glycol ; ou leurs mélanges.

**9.** Article absorbant (20) selon la revendication 6, dans lequel l'agent indiquant l'humidité est choisi dans le groupe constitué d'éthyle rouge, de bromophénol bleu, de bromocrésol vert, ou de résazurine.

**10.** Article absorbant (20) selon la revendication 6, dans lequel l'indicateur d'humidité (54) est sous la forme d'un adhésif thermofusible et inclut en outre un plastifiant et un agent poisseux.

FIG. 1

FIG. 2

FIG.3

FIG. 4

**EP 1 242 027 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0813850 A
- US 3952746 A, Summers
- US 4022211 A, Timmons
- US 4192311 A, Felfoldi
- US 4287153 A, Towsend
- US 4705513 A, Sheldon
- US 5354289 A, Mitchell
- US 5690624 A, Sasaki
- US 5897541 A, Uitenbroek
- JP 3221039 A
- JP 10075980 A
- US 5066711 A, Colon
- US 4895567 A, Colon
- US 4743238 A, Colon
- US 4681576 A, Colon
- US 5342861 A, Raykovitz
- US 5766212 A, Jitoe
- JP 10085257 A
- US 3860003 A
- US 5151092 A
- US 4610678 A
- US 4673402 A
- US 4888231 A
- US 4834735 A
- US 5234423 A
- US 5147345 A
- US 4988344 A
- US 4988345 A
- US 3929135 A
- US 4324246 A
- US 4342314 A
- US 4463045 A
- US 5006394 A
- US 4573986 A
- US 3911173 A, Sprague, Jr.
- US 4785996 A, Ziecker
- US 4842666 A, Werenicz
- US 4857067 A
- US 4381781 A, Sciaraffa
- US 4938753 A, Van Gompel
- US 4909803 A, Aziz
- US 4695278 A, Lawson
- US 4795454 A, Dragoo
- US 4704115 A, Buell
- US 4515595 A
- US 3848594 A, Buell
- US 4662875 A, Hirotsu and Robertson
- US 4869724 A, Scripps
- US 4846815 A, Scripps
- US 4894060 A, Nestegard
- US 4946527 A, Battrell
- US 5326612 A